# EUROPEAN PATENT APPLICATION

(11) **EP 4 032 982 A1**
(43) Date of publication of application: **27.07.2022**
(21) Application number: 20835401.9
(22) Date of filing: 25.06.2020
(51) Int. Cl.: C12N 15/79, C12N 15/67, C12P 21/00, C07K 7/08

(54) **NOVEL PROMOTER HASP1 OF PHAEODACTYLUM TRICORNUTUM AND SIGNAL PEPTIDE THEREOF, AND USE THEREOF**

(30) Priority: 01.07.2019 KR 20190079014
(71) Applicant: Korea Institute of Science and Technology, Seoul 02792 (KR); Algaeprona Inc., Gangwon-do 25451 (KR)
(72) Inventor: PAN, Cheol-Ho, Gangneung-si, Gangwon-do 25451 (KR); ERDENEDOLGOR, Erdene-Ochir, Gangneung-si, Gangwon-do 25451 (KR); SONG, Dae-geun, Gangneung-si, Gangwon-do 25451 (KR); SHIN, Bok-Kyu, Gangneung-si, Gangwon-do 25537 (KR); KWON, Byeori, Gangneung-si, Gangwon-do 25490 (KR)
(74) Representative: Gilani, Anwar
(86) International application number: PCT/KR2020/008251
(87) International publication number: WO 2021/002630

(57) **Abstract**

The present invention relates to a novel promoter *HASP1* for inducing overexpression of a protein in *Phaeodactylum tricornutum,* a signal peptide HASP1-SP, which is a novel protein secretion sequence for inducing the secretion of the expressed protein, an expression vector including the promoter *HASP1* and the HASP1-SP-encoding sequence, a transformant in which the expression vector has been introduced into a host cell, a method for producing a protein of interest including a step of culturing the transformant to obtain a culture, a method for expressing a protein of interest including a step of transforming a host cell with the expression vector, and a method for increasing an expression level of a protein of interest in a host cell including a step of transforming the host cell with the expression vector. The novel promoter *HASP1* for inducing overexpression of a protein in *Phaeodactylum tricornutum,* the signal peptide HASP1-SP, which is a novel protein secretion sequence for inducing the secretion of the expressed protein, and the expression vector including the promoter HASP1 and the HASP1-SP-encoding sequence may be used as strong genetic tools capable of significantly increasing productivity of recombinant proteins.

## Description

### [Technical Field]

The present invention relates to a novel promoter *HASP1* of microalga *Phaeodactylum tricornutum,* a signal peptide thereof, and a use thereof, and more particularly to a novel promoter *HASP1* for driving overexpression of a protein in *Phaeodactylum tricornutum,* a signal peptide HASP1-SP, which is a novel protein secretion sequence for inducing secretion of the expressed protein, an expression vector including the *HASP1* and the HASP1-SP, a transformant in which the expression vector has been introduced into a host cell, a method for producing a protein of interest including a step of culturing the transformant to obtain a culture, a method for expressing a protein of interest including transforming a host cell with the expression vector, and a method for increasing an expression level of a protein of interest in a host cell including transforming the host cell with the expression vector.

### [Background Art]

Microalgae have drawn attention as a bio-factory for producing high-value-added natural substances and functional proteins. Among microalgae, diatoms are unicellular eukaryotic phytoplankton that inhabit marine and freshwater environments and are responsible for about 20% of the primary photosynthetic productivity on Earth. Compared with other microalgae, diatoms have a higher carbon fixation ability.

Diatoms are being actively studied for diverse biotechnological applications and may be used as bioreactors for production of biopharmaceuticals and secondary metabolites.

Among diatoms, *Phaeodactylum tricornutum* is a coastal marine diatom that utilizes silicon. The complete genome of *Phaeodactylum tricornutum* is approximately 27.6 Mb in size and consists of 33 chromosomes containing 12,177 genes. A large number of expressed sequence tags (ESTs) have been identified in *Phaeodactylum tricornutum* cells grown under various different conditions including different sources of nutrients, lighting sources, and abiotic stresses. Furthermore, research has been conducted on tools for genetic manipulation of *Phaeodactylum tricornutum,* such as gene overexpression, gene silencing, gene editing, and plasmid delivery methods.

Specifically, a pPha-T1 vector was first developed as a tool for manipulating genes for transformation of *Phaeodactylum tricornutum* (Apt, Grossman *et al.,* 1996). In the pPha-T1 vector, a photosynthesis-associated fucoxanthin chlorophyll a binding protein (*fcpA*) promoter is used. In addition, an expression vector using a nitrate reductase (NR) promoter that is expressed in nitrate deficiency has been developed (Poulsen and Kroger, 2005).

However, both of the promoters have a disadvantage in that expression of proteins maintained at high levels during the exponential phase in the growth phases significantly decreases in the stationary phase, and therefore there is still absence of a promoter which strongly drives overexpression of recombinant proteins in microalgae.

Meanwhile, it is essential to induce secretion of the proteins after protein overexpression for efficiency of a purification process, and thus suitable secretion signal peptides are required.

Although ARS1 of *Chlamydomonas reinhardtii* has been reported as a secretion peptide in microalgae (Ramos *et al.,* 2017), it is only known that expressed human IgG antibodies are secreted into a culture medium of *Phaeodactylum tricornutum* (Hempel *et al.,* 2012), and secretion peptides have rarely been studied.

Therefore, there is also a need to develop a signal peptide inducing secretion of expressed proteins in order to express a protein from microalgae and to increase efficiency of the purification process.

### [Disclosure]

### [Technical Problem]

As a result of intensive efforts to develop novel expression systems for producing recombinant proteins with high yields in a microalga *Phaeodactylum tricornutum,* the present inventors have developed a novel promoter, *HASP1,* which strongly drives overexpression of recombinant proteins in *Phaeodactylum tricornutum,* and a signal peptide, HASP1-SP, which efficiently promotes secretion of the recombinant proteins into a culture medium, and have confirmed that productivity of the recombinant proteins is increased by strongly driving expression of the recombinant proteins throughout all growth phases, especially during the stationary phase, thereby completing the present invention.

### [Technical Solution]

An object of the present invention is to provide an *HASP1* promoter including a polynucleotide of SEQ ID NO: 1.

Another object of the present invention is to provide an HASP1 signal peptide encoded by a polynucleotide of SEQ ID NO: 2.

Another object of the present invention is to provide an expression vector including the *HASP1* promoter and/or the HASP1 signal peptide.

Another object of the present invention is to provide a transformant in which the expression vector has been introduced into a host cell.

Another object of the present invention is to provide a method for producing a protein of interest including a step of culturing the transformant to obtain a culture and recovering the protein of interest from the culture.

Another object of the present invention is to provide a method for expressing a protein of interest including a step of transforming a host cell with the expression vector.

Another object of the present invention is to provide a method for increasing an expression level of a protein of interest in a host cell including a step of transforming the host cell with the expression vector.

Another object of the present invention is to provide a use of the promoter and/or the signal peptide for increasing an expression level of a protein of interest in a host cell.

### [Advantageous Effects]

The novel promoter *HASP1* for driving overexpression of a protein in *Phaeodactylum tricornutum,* the signal peptide HASP1-SP, which is a novel protein secretion sequence for inducing secretion of the expressed protein, and the expression vector including the HASP1 and the HASP1-SP according to the present invention are expected to be used as strong genetic tools for significantly increasing productivity of recombinant proteins.

### [Brief Description of Drawings]

FIG. 1 shows SDS-PAGE results for protein analysis in cultures of *Phaeodactylum tricornutum.*
FIG. 2 shows a sequence of HASP1 protein present in a culture medium of *Phaeodactylum tricornutum* and identified by LC-MS/MS.
FIG. 3 shows locations of an *HASP1* promoter and a signal peptide sequence on genomic DNA of *Phaeodactylum tricornutum.*
FIG. 4 shows an expression vector including the *HASP1* promoter or both the *HASP1* promoter and the signal peptide.
FIG. 5 shows actions of the *HASP1* promoter and the signal peptides during growth phases of *Phaeodactylum tricornutum.* FIG. 5A shows expression levels of GFP in cells with respect to growth phases, and FIG. 5B shows expression levels of GFP secreted into culture media with respect to growth phases.
FIG. 6 shows expressions of GFP with respect to incubation periods and locations where GFP is expressed in the presence and absence of a signal peptide obtained by a fluorescent microscope. FIG. 6A shows results of a control in which GFP is not expressed, FIG. 6B shows GFP expressed by an *fcpA* promoter, FIG. 6C shows GFP expressed by an *HASP1* promoter, and FIG. 6D shows GFP expressed in the presence of an HASP1 signal peptide.

### [Best Mode]

The present invention will be described in detail. Meanwhile, each description and embodiment disclosed in the present invention may be applied herein to describe different descriptions and embodiments. In other words, all combinations of various components disclosed in the present invention are included within the scope of the present invention. Furthermore, the scope of the present invention should not be limited by the detailed description provided below.

Also, those skilled in the art will recognize or be able to ascertain, using no more than routine experimentation, many equivalents to specific embodiments of the present invention. Such equivalents are intended to be encompassed in the scope of the following claims.

An aspect of the present invention to achieve the above-described objects provides an *HASP1* promoter for driving overexpression of a protein of interest in microalgae, including a polynucleotide having a nucleotide sequence of SEQ ID NO: 1 below.

SEQ ID NO: 1

5'-catacagtga atgtaacttt cgaattgaca gtattagtag tcgtattgac agtgaggcac
gcccctcaat gtgcgaggtg gaaaatatac cagcatgaca atgaatcttg gagattcttt
tgctgtcatc aagattcacc gccaaatctt caggaaccta tcacgtccac aggcgatgtt
aattcttgag tcgtcaaaac aaagtcctgt cctacctgta gaagttgaca gcgagcaatt
gtatgcaaac ttctgacttt gttataataa cattaaaggt aattaagtat cttcaattag
gcattttgtc actgtcagtc cgttccgaca atataggtag atttggaatg aatcttttct
atgctgctgc gaatcttgta cacctttgag gccgtagatt ctgtccgacg aagcgataat
tattgcaaaa tacatggact cattattttg attcgatttc tttttggtat ccgactcgaa
aagatccatc acggcgagc-3'

As used herein, the term "HASP1" refers to a highly abundant secreted protein 1 (HASP1) detected in a microalga *Phaeodactylum tricornutum* with a high content and refers to a protein consisting of 1,793 amino acids containing a phytase-like domain in the C-terminal region and belonging to the phytase superfamily.

As used herein, the term "microalgae" refers to phytoplankton present in water and performing photosynthesis. About 500,000 species exists in marine or freshwater systems and live in extreme environments such as hot springs and the deep sea. The microalgae have a high degree of utilization as a biofuel and a functional protein.

As used herein, the term *"Phaeodactylum tricornutum"* is a coastal marine diatom that utilizes silicon. The diatoms are unicellular eukaryotes that inhabit marine and freshwater environments and are microalgae having a high carbon fixation ability to the extent that the diatoms are responsible for about 20% of the primary photosynthetic productivity on Earth. The complete genome of *Phaeodactylum tricornutum* is approximately 27.6 Mb in size and consists of 33 chromosomes containing 12,177 genes.

As used herein, the term "promoter" refers to a polynucleotide that allows and regulate transcription of a gene operably linked thereto. The promoter includes a recognition sequence for binding to an RNA polymerase and a transcription initiation site. The promoter is selected to express a protein of interest in a particular cell type or a host cell. That is, the promoter is an untranslated upstream nucleic acid sequence of an encoded region, i.e., a DNA region to which a polymerase binds to initiate transcription of the gene, by including a polymerase-binding site and having activity to initiate transcription of downstream genes into mRNA. The promoter is located at a 5'-region of an mRNA transcription initiation region.

As used herein, the term *"HASP1* promoter" refers to a promoter derived from *Phaeodactylum tricornutum,* including a polynucleotide having a nucleotide sequence of SEQ ID NO: 1 and driving overexpression of a protein of interest in microalgae. The promoter is present in the 499 bp upstream region before a start codon of the *HASP1* gene. The *HASP1* gene is a gene encoding HASP1 protein (PHATRDRAFT_47612), and a nucleotide sequence thereof may be obtained from a known database such as GenBank of the NCBI (NCBI accession number: XM_002181840.1 ).

The *HASP1* promoter of the present invention is a novel promoter first identified in *Phaeodactylum tricornutum.*

Any nucleotide sequence having a homology of 85% or more, specifically 90% or more, more specifically 95% or more, even more specifically 98% or more, and most specifically 99% or more with the above-described nucleotide sequence may be used without limitation, as long as the nucleotide sequence may act as a promoter equivalent or substantially identical to a nucleic acid molecule consisting of the polynucleotide. Also, it is obvious that a nucleotide sequence including deletion, modification, substitution, or addition of one or more nucleotides falls within the scope of the present invention, as long as it has the above-described homology.

As used herein, the term "homology" refers to a percentage of identity between two polynucleotide or polypeptide moieties. A sequence homology of one moiety with another moiety may be determined using technologies known in the art. For example, the homology may be determined by directly aligning sequence information, for example, parameters such as score, identity, and similarity, between two polynucleotide molecules or two polypeptide molecules using a computer program that aligns sequence information and is commercially available (*e.g*.: BLAST 2.0). In addition, homology between polynucleotides may be determined by hybridizing the polynucleotides under the condition of forming stable double strands between homologous regions, digesting the double strands using a single-strand-specific nuclease, and measuring the size of digested fragments.

As used herein, the term "protein of interest" refers to a protein, expression level of which is intended to be specifically and increasingly induced in microalgae by using the *HASP1* promoter provided in the present invention in a state being operably linked to the *HASP1* promoter. Specifically, the protein of interest may be a protein present in the microalgae or a protein produced by the microalgae but is not limited thereto and may be appropriately selected by those of ordinary skill in the art in accordance with the objects of the present invention. More specifically, the protein of interest may be a protein, expression of which is specifically and increasingly induced in diatoms, even more specifically a protein, expression of which is specifically and increasingly induced in *Phaeodactylum tricornutum.*

In the present invention, the "protein of interest" may be used interchangeably with "product of interest".

The *Phaeodactylum tricornutum-derived HASP1* promoter may provide effects on inducing an increase in expression of the protein of interest in microalgae. Such effects of the promoter were first identified by the present inventors.

In a specific embodiment of the present invention, it was confirmed that expression of GFP was significantly increased when *Phaeodactylum tricornutum* was transformed with an expression vector including the *HASP1* promoter and the protein of interest GFP, compared to a control (Example 5).

Another aspect of the present invention provides an HASP1 signal peptide expressed by a polynucleotide having a nucleotide sequence of SEQ ID NO: 2 or composed of an amino acid sequence of SEQ ID NO: 3.
SEQ ID NO: 2
   5'-accatgaatc ttcgttgtat ccttccgttt ctcctcgcaa gcttctcggc tggg-3'
SEQ ID NO: 3
   N-MNLRCILPFLLASFSAGA-C

The "HASP1" of the present invention is as described above.

As used herein, the term "signal peptide" refers to a particular region located at the N-terminus of a secreted protein or a membrane protein and including 15 to 20 amino acids and acting as a signal when a protein passes through a membrane.

As used herein, the term "HASP1 signal peptide" refers to an 18-amino acid long protein responsible for secretion of HASP1 and encoded by a polynucleotide of SEQ ID NO: 2 which is 54 bp long in the 3' direction of the *HASP1* gene.

Any nucleotide sequence having a homology of 85% or more, specifically 90% or more, more specifically 95% or more, even more specifically 98% or more, and most specifically 99% or more, with the sequence may be included without limitation, as long as it acts as a signal peptide equivalent or substantially identical to a nucleic acid consisting of the polynucleotide. Also, it is obvious that a nucleotide sequence including deletion, modification, substitution, or addition of one or more nucleotides falls within the scope of the present invention, as long as it has the above-described homology.

The "homology" of the present invention is as described above.

In a specific embodiment of the present invention, it was confirmed that the HASP1 signal peptide efficiently induced introduction of GFP protein, which is a protein of interest (Example 6).

Another aspect of the present invention provides an expression vector including the *HASP1* promoter of SEQ ID NO: 1 and/or the HASP1 signal peptide-encoding sequence of SEQ ID NO: 2.

The "HASP1", "promoter", *"HASP1* promoter", "signal peptide", and "HASP1 signal peptide" of the present invention are as described above.

As used herein, the term "expression vector" refers to an artificial DNA molecule including a genetic material to express a target gene in a proper host cell, specifically a DNA construct including a nucleotide sequence of a gene operably linked to an appropriate regulatory sequence. The regulatory sequence may include a promoter capable of initiate transcription, any operator sequence for regulating the transcription, a sequence encoding a suitable mRNA ribosome binding site, and a sequence for regulating termination of transcription and translation, without being limited thereto. The promoter of the present invention may be the *HASP1* promoter.

In view of the objects of the present invention, the recombinant expression vector may include a foreign gene operably linked to the promoter and encoding the protein of interest.

As used herein, the term "foreign gene" refers to a gene introduced into a plant from the outside and may already be or may not be present in an individual (a target of transformation). The nucleotide sequence of the foreign gene may be obtained by codon optimization of the protein of interest.

As used herein, the term "operably linked" means a functional linkage between the gene sequence and the promoter sequence such that the nucleic acid sequence having the promotor activity of the present invention initiates and mediates transcription of the gene encoding the protein of interest. The operable linkage may be prepared using a genetic recombination technique known in the art, and site-specific DNA ligation may be performed using a ligase known in the art, without being limited thereto.

The vector used in the present invention is not particularly limited as long as the vector replicates in host cells, and any vector known in the art may be used. Examples of the vector known in the art may include a natural or recombinant plasmid, cosmid, virus, and bacteriophage. For example, pWE15, M13, AMBL3, AMBL4, λIXII, λASHII, λAPII, λt10, λt11, Charon4A, and Charon21A may be used as the phage vector or the cosmid vector. As the plasmid vector, pBR type, pUC type, pBluescriptll type, pGEM type, pTZ type, pCL type, and pET type may be used. The vector available in the present invention is not particularly limited, and any known expression vector may be used. For example, pDZ, pACYC177, pACYC184, pCL, pECCG117, pUC19, pBR322, pMW118, pCC1BAC, and pCES208 vectors, specifically pPha vector, may be used, without being limited thereto.

In addition, an endogenous promoter in the chromosome may be substituted with a nucleic acid molecule having the promoter activity of the present invention using a vector for chromosomal insertion in a host cell. The insertion of the nucleic acid molecule into the chromosome may be performed by way of any method known in the art, e.g., homologous recombination. Since the vector of the present invention may be inserted into the chromosome by homologous recombination, the nucleic acid may further include a selection marker to confirm chromosomal insertion. The selection marker is used to select cells that are transformed with the vector, that is, to confirm insertion of a target nucleic acid molecule, and markers providing selectable phenotypes such as drug tolerance, nutrient requirement, resistance to cytotoxic agents, or expression of surface proteins may be used. Since only cells expressing the selection marker are able to survive or show different phenotypes under the environment treated with a selective agent, transformed cells may be selected.

The expression vector of the present invention may be an expression vector including the *HASP1* promoter or an expression vector including the *HASP1* promoter and the HASP1 signal peptide-encoding sequence.

Another aspect of the present invention provides a transformant in which the expression vector has been introduced into a host cell.

The "expression vector" of the present invention is as described above.

As used herein, the term "transformation" refers to a process of introducing a vector including a polynucleotide encoding a protein of interest into a host cell in such a way that the protein encoded by the polynucleotide is expressed in the host cell.

The transformed polynucleotide may be either in a form inserted into the chromosome of the host cell or in a form located outside the chromosome, as long as the protein is expressed in the host cell. In addition, the polynucleotide includes DNA and RNA encoding the protein of interest. The polynucleotide may be introduced into the host cell in any form as long as the polynucleotide is introduced into the host cell and the protein is expressed therein. For example, the polynucleotide may be introduced into the host cell in the form of an expression cassette, which is a gene construct including all of the essential elements required for self-replication. The expression cassette may generally include a promoter operably linked to the polynucleotide, a transcription termination signal, a ribosome binding site, and a translation termination signal. In view of the objects of the present invention, the promoter may include a nucleic acid molecule having the activity of the promoter having a nucleotide sequence of SEQ ID NO: 1 of the present invention. The expression cassette may be in the form of a self-replicable expression vector. Also, the polynucleotide may be introduced into the host cell in its original form and operably linked to a sequence required for the expression in the host cell.

Methods for transforming with the vector according to the present invention include any methods enabling introduction of a nucleic acid into a host cell and may be performed by suitable standard techniques well known in the art selected according to the host cell. For example, particle bombardment, electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method may be used, without being limited thereto.

The "host cell" of the present invention refers to a microorganism into which a nucleic acid having the promoter activity of the present invention is introduced and operates as a promoter. In a specific embodiment of the present invention, the microorganism may be a microalga, specifically a strain belonging to the genus *Phaeodactylum* sp., and more specifically *Phaeodactylum tricornutum,* without being limited thereto.

As used herein, the term "transformant" refers to a microorganism transformed by introducing the expression vector including the *HASP1* promoter and/or the HASP1 signal peptide and the protein of interest into the host cell *Phaeodactylum tricornutum.*

In a specific embodiment of the present invention, overexpression of the protein of interest GFP was confirmed in the transformant during the stationary phase among the cell growth phases (Example 5).

Another aspect of the present invention provides a method for producing a protein of interest, the method including steps of culturing the transformant to obtain a culture, and recovering the protein of interest from the culture.

The "transformant" and "protein of interest" of the present invention are as described above.

As used herein, the term "culturing" refers to growing the microorganism in an artificially adjusted appropriate environment. In the present invention, the method for preparing the product of interest using the microorganism belonging to the genus *Phaeodactylum* sp. may be performed using any methods well known in the art. Specifically, the culturing may be performed continuously by a batch process, a fed-batch process, or a repeated fed-batch process, without being limited thereto.

A culture medium used for the culturing of the present invention needs to satisfy requirements of a particular strain in an appropriate method. Examples of an available carbon source may include: saccharides and carbohydrates such as glucose, sucrose, lactose, fructose, maltose, starch, and cellulose, oils and fats such as soybean oil, sunflower oil, castor oil, and coconut oil, fatty acids such as palmitic acid, stearic acid, and linoleic acid, alcohols such as glycerol and ethanol, and organic acids such as gluconic acid, acetic acid, and pyruvic acid, without being limited thereto. These substances may be used alone or in combination.

Examples of an available nitrogen source may include peptone, yeast extract, meat gravy, malt extract, corn steep liquor, bean flour, and urea, or an inorganic compound such as ammonium sulfate, ammonium chloride, ammonium phosphate, ammonium carbonate, and ammonium nitrate, without being limited thereto. These nitrogen sources may also be used alone or in combination.

Examples of an available phosphorous source may include potassium dihydrogen phosphate, dipotassium hydrogen phosphate, or sodium-containing salts corresponding thereto, without being limited thereto. In addition, the culture medium may include materials required for growth such as a metal salt such as magnesium sulfate or iron sulfate. Furthermore, essential growth-promoting ingredients such as amino acids and vitamins may be added thereto. In addition, suitable precursors may be used in the culture medium. The above-described raw ingredients may be appropriately added to the culture during the culturing process in a batch or continuous manner.

The pH of the culture may be adjusted by appropriately using a basic compound such as sodium hydroxide, potassium hydroxide, or ammonia or an acidic compound such as phosphoric acid or sulfuric acid. Also, a defoaming agent such as fatty acid polyglycol ester may be added during culturing in order to inhibit formation of foams. Oxygen or oxygen-containing gas may be injected into the culture to maintain an aerobic condition. The temperature of the culture may be maintained at 20°C to 45°C, preferably at 25°C to 40°C, but may vary according to conditions, without being limited thereto.

The method for producing a product of interest of the present invention may include recovering the product of interest from a microorganism or a culture. As used herein, the term "recovering" is collecting the product of interest from the microorganism or the culture using any method known in the art, *e.g.,* centrifugation, filtration, anion exchange chromatography, crystallization, and high-performance liquid chromatography (HPLC), without being limited thereto.

The step of recovering may include a purification process selected by those of ordinary skill in the art among a variety of purification processes, if required.

Another aspect of the present invention provides a method for expressing a protein of interest including transforming a host cell with the expression vector.

Another aspect of the present invention provides a method for increasing an expression level of a protein of interest in a host cell, including transforming the host cell with the expression vector.

The "expression vector", "host cell", "transformation", and "protein of interest" of the present invention are as described above.

In a specific embodiment of the present invention, it was confirmed that the expression level of the recombinant protein of interest GFP significantly increased when *Phaeodactylum tricomutum* is transformed with the expression vector including the *HASP1* promoter and/or HASP1-SP signal peptide, compared to a control. Also, the increase in the expression level of the protein of interest was confirmed even in the stationary phase as well as the exponential phase among the cell growth phases (Examples 5 and 6).

Therefore, the expression vector including the *HASP1* promoter and/or a signal peptide thereof according to the present invention may be used as a strong tool for efficient expression and secretion of the recombinant protein throughout all growth phases of *Phaeodactylum tricornutum.*

Another aspect of the present invention provides a use of the promoter or the signal peptide for increasing an expression level of a protein of interest in a host cell.

### [Mode for Invention]

Hereinafter, the present invention will be described in more detail with reference to the following examples. However, the following examples are merely presented to exemplify the present invention, and the scope of the present invention is not limited thereto.

### Example 1. Selection and Identification of Protein Secreted from Phaeodactylum tricornutum

A microalga of the present invention, *i.e., Phaeodactylum tricornutum,* was obtained from the UTEX Culture Collection of Algae (The University of Texas at Austin, TX, USA). The *Phaeodactylum tricornutum* strain was inoculated at an initial concentration of 1×10⁵ cells/mL and cultured in 200 mL of an F/2 medium containing 50% autoclaved seawater.

When *Phaeodactylum tricornutum* were grown to the stationary phase in the growth phase, *i.e.,* on the 5^{th} or 6^{th} day, the culture was centrifuged at 20°C for 15 minutes at 3500 rpm to remove cell pellets, and the remaining culture medium was concentrated using a centrifugal filter (Vivaspin, Satorius, Goettingen, Germany) provided with a 5 kDa cut-off membrane.

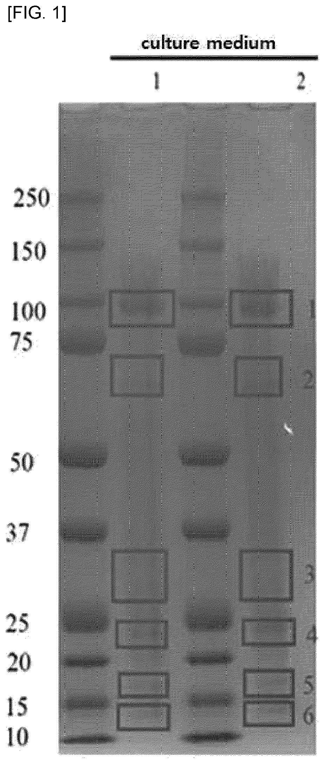

The proteins were resolved by electrophoresis on 12% SDS-PAGE gel and visualized by Coomassie blue staining (FIG. 1). The isolated proteins were excised from the gel and obtained by in-gel digestion.

Subsequently, the products were analyzed by LC-MS/MS (LTQ XL linear ion trap mass spectrum, Thermo Scientific) using a C18 trap column (2.5 cm × 100 µm, Upchurch Scientific) and a homemade reverse phase C18 micro column (10 cm × 100 µm, Nanobaume).

MS/MS data analysis was performed using Sorcerer-SEQUEST (version 3.5) and protein search was performed using UniProt *P. tricornutum* protein database.

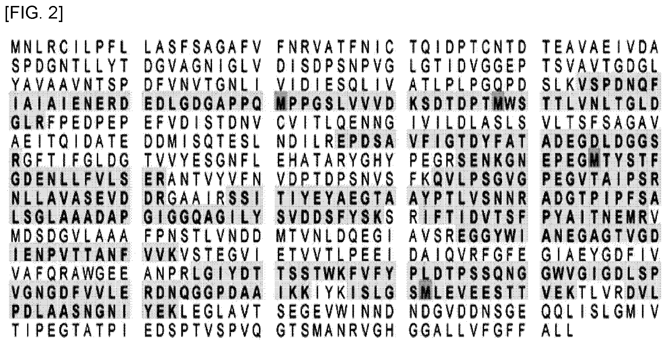

As a result, 468 proteins were identified, and the most abundant secreted protein was PHATRDRAFT_47612 (NCBI accession number: XM_002181840.1) identified through database searching with a 44% sequence coverage (FIG. 2). The protein is HASP1 (highly abundant secreted protein 1) consisting of 1,793 amino acids and belongs to the phytase superfamily containing a phytase-like domain at the C-terminus.

The level of HASP1 protein was significantly higher than those of other proteins in the culture medium (Table 1).

**Table 1**

| **#** | **Description** | **Uniprot accession number** | **Molecular weight** | **Spectru m counts** | **Note** |
|---|---|---|---|---|---|
| 1 | Predicted protein OS=*Phaeodactylum tricornutum* (strain CCAP 1055/1) GN=PHATRDRAFT_47612 PE=4 SV=1 | B7G4A0_PHA TC | 84 kDa | 1811 | **HASP1** |
| 2 | Predicted protein OS=*Phaeodactylum tricornutum* (strain CCAP 1055/1) GN=PHATR_46677 PE=4 SV=1 | B5Y3F2_PHA TC | 51 kDa | 245 | |
| 3 | Predicted protein OS=*Phaeodactylum tricornutum* (strain CCAP 1055/1) GN=PHATRDRAFT_43513 PE=4 SV=1 | B7FSH1_PHA TC | 49 kDa | 211 | |
| 4 | Endo-1,3-beta-glucosidase OS=*Phaeodactylum tricornutum* (strain CCAP 1055/1) GN=PHATRDRAFT_54681 PE=4 SV=1 | B7G259_PHA TC | 112 kDa | 201 | |
| 5 | Predicted protein *OS=Phaeodactylum tricornutum* (strain CCAP 1055/1) GN=PHATRDRAFT_49678 PE=4 SV=1 | B7GBF3_PHA TC | 79 kDa | 157 | |

Therefore, the HASP1 protein was selected, and a potential promoter region thereof and a putative signal peptide having effects on production and secretion of recombinant proteins in *Phaeodactylum tricornutum* were isolated. In addition, the *fcpA* promoter, which is a widely used promoter in *Phaeodactylum tricornutum,* was used as a control.

### Example 2. Isolation of Promoter HASP1 and Signal Peptide HASP1-SP from Phaeodactylum tricornutum and Construction of Vector Including the Same

The *Phaeodactylum tricornutum* genome has a relatively high gene density compared to other higher eukaryotic genomes. Approximately, 500 bp endogenous genes were sufficient for full promoter activity by strongly driving transgene expression.

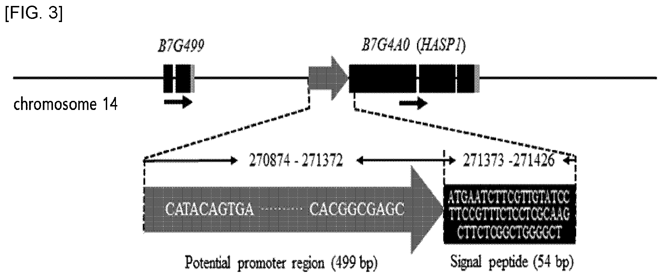

Therefore, a 499 bp upstream region before the start codon of the *HASP1* gene was selected as a potential promoter (FIG. 3).

Also, using SignalP software, a 54 bp 3' region of the *HASP1* gene, *i.e.,* an 18-amino-acid-long signal peptide responsible for HASP1 secretion was selected (FIG. 3).

Subsequently, 50 mL of the culture medium of *Phaeodactylum tricornutum* cultivated in an F/2 medium containing 50% artificial seawater was centrifuged at 20°C at 3500 rpm for 15 minutes to obtain cell pellets, and genomic DNA (gDNA) was extracted therefrom.

Genes were amplified from gDNA of *Phaeodactylum tricornutum* by PCR using HASP1-F and HASP1-R primers, and pfu polymerase, and the *fcpA* promoter of the pPha-T1 vector was replaced using Ndel and EcoRI restriction enzymes to construct a pPha-HASP1p vector.

The *GFP* gene was amplified from a pEGFP-C2 vector by PCR using GFP-F and GFP-R primers, and the *GFP* gene was inserted next to the *HASP1* promoter using Nhel and BamHI restriction enzymes to construct a pPha-HASP1p-GFP vector.

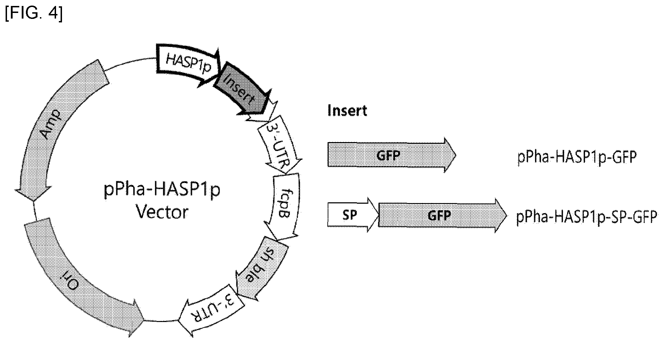

The gene of the HASP1 signal peptide (HASP1-SP) was synthesized and inserted between the promoter and the *GFP* gene of the pPha-HASP1p-GFP vector using EcoRI and Nhel restriction enzymes to construct a pPha-HASP1p-SP-GFP vector (FIG. 4).

For comparison of characteristics between the *HASP1* promoter and the *fcpA* promoter, a pPha-T1-GFP vector and a pPha-T1-SP-GFP vector were constructed using the pPha-T1 vector according to the above-described method.

Primers used to construct the vectors and synthesized genes are shown in Table 2 below.

**Table 2**

| **#** | **Name** | **Sequence** | **Restriction enzyme** | **Annotation** |
|---|---|---|---|---|
| 1 | HASP1-F | CATATGCATACAGTGAATGTAA CTTTCG | Ndel | pPha-T1-HASP1; PCR and Genomic-DNA PCR |
| 2 | HASP1-R | GAATTCGCTCGCCGTGATGGA TCTTTTC | EcoRI | pPha-T1-HASP1; PCR |
| 3 | GFP-Nhel-F | GCTAGCATGGTGAGCAAGGGC GAGGAG | | |
| 4 | GFP-EcoRI-F | GAATTCATGGTGAGCAAGGGC GAGGAG | Nhel/EcoRI/ Ndel | pPha-T1-*gtp*; PCR |
| 5 | GFP-R | GGATCCTTACTTGTACAGCTCG TCCATGC | BamHI | pPha-T1-*gtp*; PCR |
| 6 | Kozak-SP-GFP | GAATTCACCATGAATCTTCGTT GTATCCTTCCGTTTCTCCTCGC AAGCTTCTCGGCTGGGGCTAG | EcoRI-NheI | Long primer for SP amplification; PCR |
| | | CATGGTGAGCAAGGGCGAGGA G | | |
| 7 | pPha-Up-Ndel-F | GTGTCGGGGCTGGCTTAAC | | Genomic DNA PCR |
| 8 | pPha-Down-MCS-R | GCCGCACGTCCCTGGTTG | | Genomic DNA PCR |

### Example 3. Transformation of Phaeodactylum tricornutum

*E*. *coli* DH5α (NEB^{®} 5-alpha Competent *E. coli*) was transformed with the vectors constructed in Example 2, and the transformed *E. coli was* cultured in an LB medium supplemented with 100 µg/mL ampicillin.

Target vectors were extracted from the cultured *E. coli* by way of a Midi-prep method and subjected to phenol/chloroform gene purification and ethanol precipitation to prepare high-purity vectors (concentration of 1.0 µg/µL) required for transformation of *Phaeodactylum tricornutum* (*P. tricornutum*).

After the vectors were coated on nanogold particles, *Phaeodactylum tricornutum* were transformed with the vectors by delivering the target vectors by particle bombardment.

The transformants were selected on F/2 agar medium containing 100 µg/mL zeocin.

### Example 4. Confirmation of Presence of Target Gene in Transformant of Phaeodactylum tricornutum

In Example 3, *Phaeodactylum tricornutum* cells were transformed into five different structures (*fcpApro:SP-GFP, HASP1 pro:SP-GFP, fcpApro:GFP, HASP1pro:GFP,* and promoterless GFP) by particle bombardment. The putative transformants were selected on the F/2 agar medium containing zeocin for 2 to 4 weeks. Then, zeocin-resistant colonies were transferred to the same medium for further growth, and their zeocin resistance was reassessed and confirmed. The presence of the transgene in the genome of the putative transformants was confirmed by gDNA-PCR using *P. tricornutum* genomic DNA as a template. More than 400 colonies were grown on zeocin-containing medium and selected therefrom.

Colonies of each transformant were cultured in 200 µL of an F/2 medium containing 50% artificial seawater for 5 days and centrifuged (at 3500 rpm, for 15 minutes, at 20°C), and the supernatant was discarded. After adding a 10 µL lysis buffer (2% Nonidet P-40) thereto, the solution was maintained for 10 minutes and used for PCR. Then, PCR was conducted using pPha-Up-Ndel-F and pPha-Down-MCS-R primers and taq polymerase.

Based thereon, it was confirmed that 70% or more of the zeocin-resistant colonies included the transgene corresponding to the genome.

### Example 5. Confirmation of Activity of HASP1 Promoter and Function of Signal Peptide HASP1-SP in Phaeodactylum tricornutum

### 5-1. Quantification of HASP1 Promoter Activity Driving GFP Expression

After the presence of the transgene in the transformants was confirmed, the transformants were inoculated onto 200 mL of an F/2 medium containing 50% artificial seawater at an initial concentration of 1.0×10⁵ cells/mL and cultured with aeration. Expression of GFP with respect to incubation days was measured using a fluorometer and a fluorescence microscope to identify expression patterns according to days of incubation. Also, amounts of GFP secreted into the culture medium were measured in the same manner.

Specifically, 4 mL of each sample was centrifuged (at 3500 rpm for 15 minutes at 20°C) to pellet the cells, and a lysis buffer (50 mM Tris-HCI (pH 8.0), 150 mM NaCl, 1 mM PMSF, 1 mM EDTA, 1% Nonidet P40, and 0.1% Tween 20) was added thereto, followed by reaction for 20 minutes. Subsequently, supernatants were obtained by centrifugation (at 3500 rpm for 15 minutes at 4°C) and concentrations of GFP therein were measured using a fluorometer (excitation 485/20 nm, emission 528/20 nm), and the products were observed using a fluorescent microscope.

After 50 mL of the cultures were centrifuged (at 3500 rpm for 15 minutes at 20°C) to obtain supernatants, the supernatants were concentrated using a centrifugal filter (Vivaspin, Satorius, Goettingen, Germany) provided with a 5 kDa cut-off membrane, and concentrations of GFP were measured by fluorescence measurement.

Transgenic *P. tricornutum* cells were inoculated and grown for 22 days with a periodic assessment of cell numbers to generate growth curves. From the growth curves of the transgenic *P. tricornutum,* it was confirmed that it took approximately 4 days and 8 days to reach the exponential phase and the stationary phase, respectively. In all transgenic lines, the cell densities exceeded 1×10⁷ cells/mL at the stationary phase.

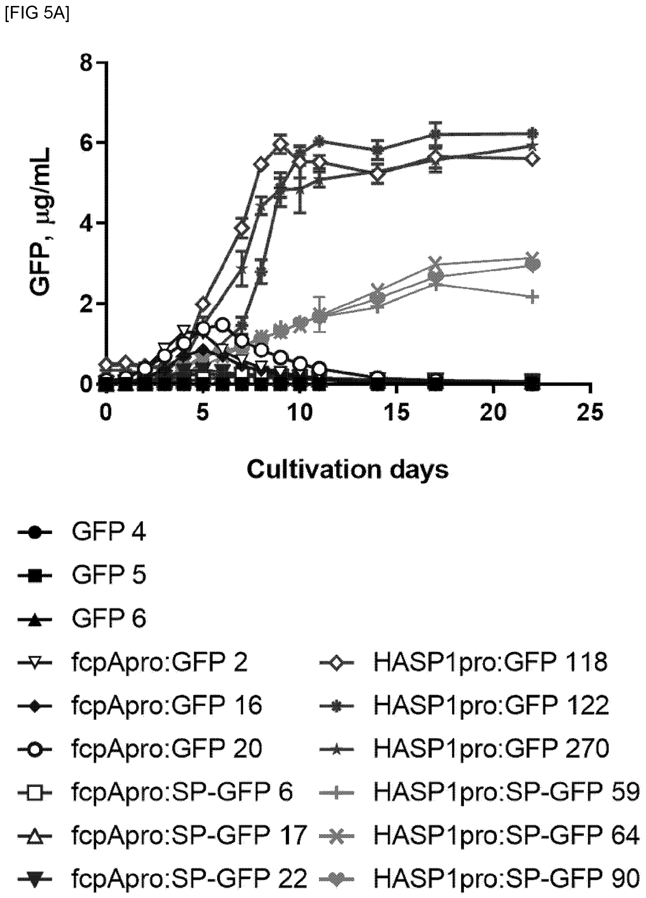

Total RNA was extracted from each transgenic cell line at both the exponential phase (day 4) and the stationary phase (day 8), and the levels of *GFP-*containing transcripts by real-time-RT-PCR to compare the activity of the *HASP1* promoter with that of the *fcpA* promoter. The levels of GFP-containing transcripts in the transgenic line expressing *HASP1pro:GFP* were 3-fold to 44-fold higher than those expressing *fcpApro:GFP* on day 4 and day 8, respectively (FIG. 5A).

Also, when compared with the promoter-less *GFP* control, the levels of GFP expression driven by the *HASP1* promoter increased by 35-fold and 754-fold on day 4 and day 8, respectively (FIG. 5A).

Meanwhile, it is well known that the *fcpA* promoter is primarily active during the exponential phase. Notably, the activity of the *HASP1* promoter was higher than that of the *fcpA* promoter during the same phase. In addition, in the case of using the *HASP1* promoter, it was confirmed that expression of GFP was maintained at a high level even during the stationary phase. Rather, the activity of the *HASP1* promoter was maintained at a higher level during the stationary phase than during the exponential phase.

On the contrary, in the case of using the *fcpA* promoter, expression of GFP increased in the exponential phase but significantly decreased in the stationary phase (FIG. 5A).

This indicates that the *HASP1* promoter may significantly increase the expression level of GFP compared to the *fcpA* promoter.

Therefore, the *HASP1* promoter may be used as a tool suitable for overexpressing recombinant proteins in *Phaeodactylum tricornutum.*

### 5-2. Confirmation of Signal Peptide HASP1-SP Efficiently Driving GFP Secretion

To investigate the role of the putative signal peptide of HASP1 in GFP secretion, fluorescent signals of intracellular secreted GFP were measured over time from the same culture of *Phaeodactylum tricornutum.*

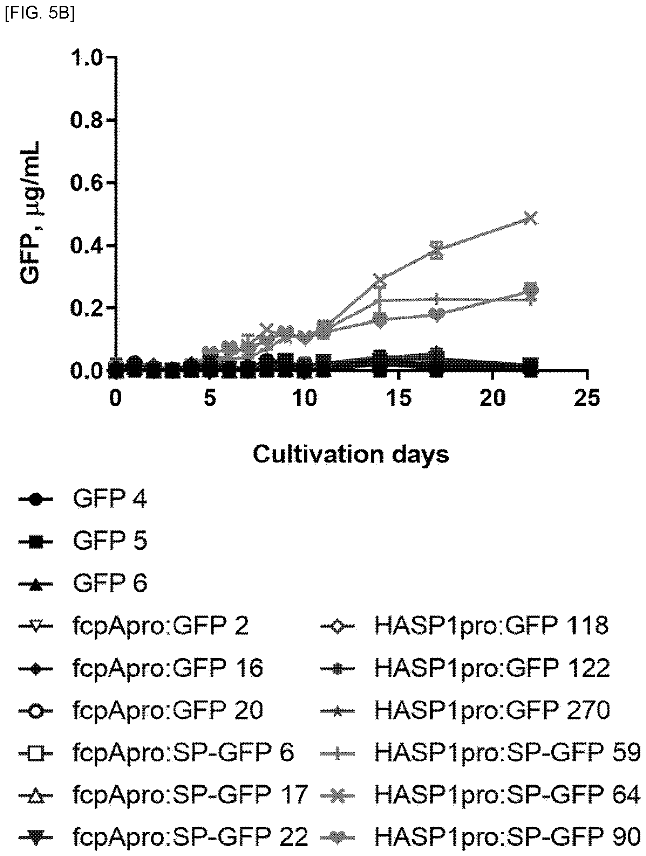

No GFP fluorescence was detected from the culture supernatants of the transgenic lines expressing *HASP1pro:GFP* or *fcpApro:GFP.* This is due to their lack of the signal peptide (FIG. 5B).

The fluorescent signal from intracellular GFP in the *HASP1pro:SP-GFP* transgenic line was similar to that of the *fcpApro:SP-GFP* transgenic line during the exponential phase but was 3-fold higher during the stationary phase. Secreted GFP levels in the *fcpApro:SP-GFP* transgenic line increased by 0.01 µg/mL during the stationary phase (FIG. 5B).

Meanwhile, secreted GFP levels in the *HASP1pro:SP-GFP* transgenic line steadily increased from the exponential phase to the stationary phase. Extracellular GFP concentrations reached 0.01 µg/mL, 0.1 µg/mL, and 0.3 µg/mL at the exponential phase, the early stationary phase, and the late stationary phase, respectively.

These values correspond to 3-fold, 8-fold, and 19-fold increases compared to that of the *fcpApro:SP-GFP* transgenic line.

Based thereon, it was confirmed that the *HASP1* promoter significantly increased GFP expression compared to the *fcpA* promoter, particularly in the stationary phase.

Also, these findings suggest that the signal peptide derived from the HASP1 protein efficiently drives GFP secretion.

### Example 6. Subcellular Localization of GFP with or without Signal Peptide of HASP1 Protein

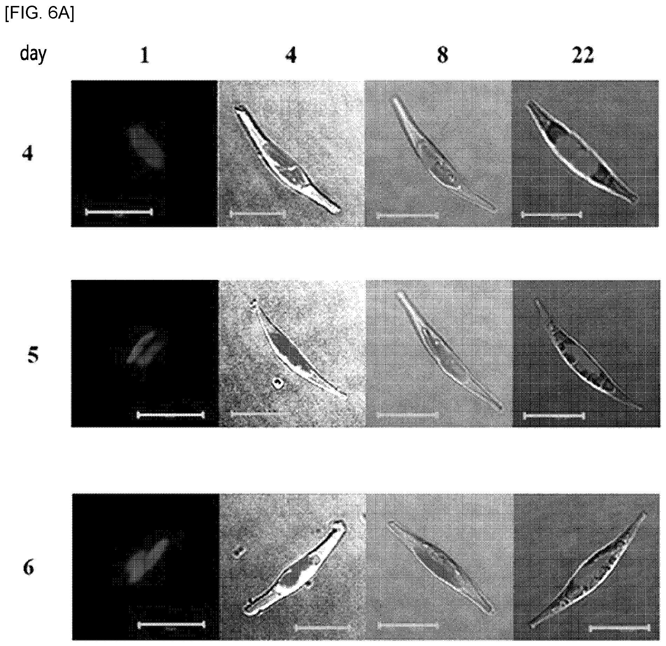

To observe the subcellular localization of GFP with or without the HASP1 signal peptide, GFP fluorescence (green) and chlorophyll autofluorescence (red) were visualized in all transgenic lines by confocal laser scanning microscopy. The transgenic line carrying the promoterless GFP was analyzed in parallel as a control (FIG. 6A).

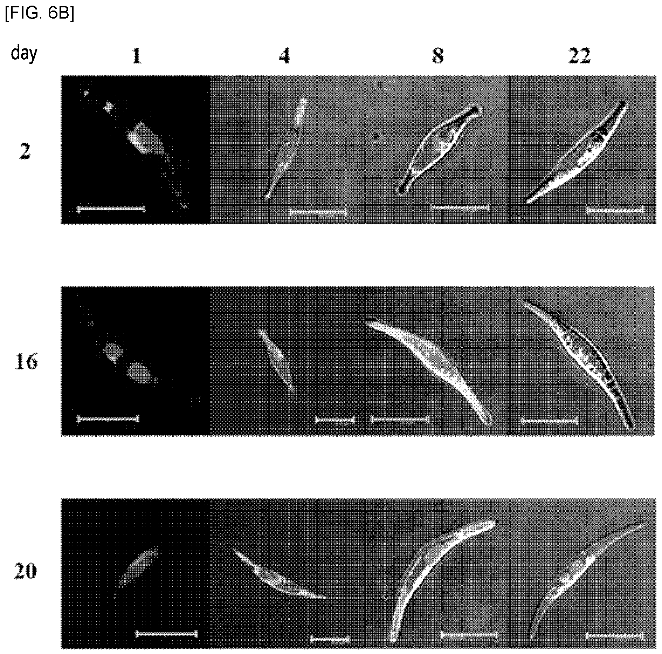

GFP fluorescence in the *fcpApro:GFP* transgenic line was diffusely localized throughout the cytoplasm at both the exponential and early stationary phases but suddenly decreased at the late stationary phase (FIG. 6B).

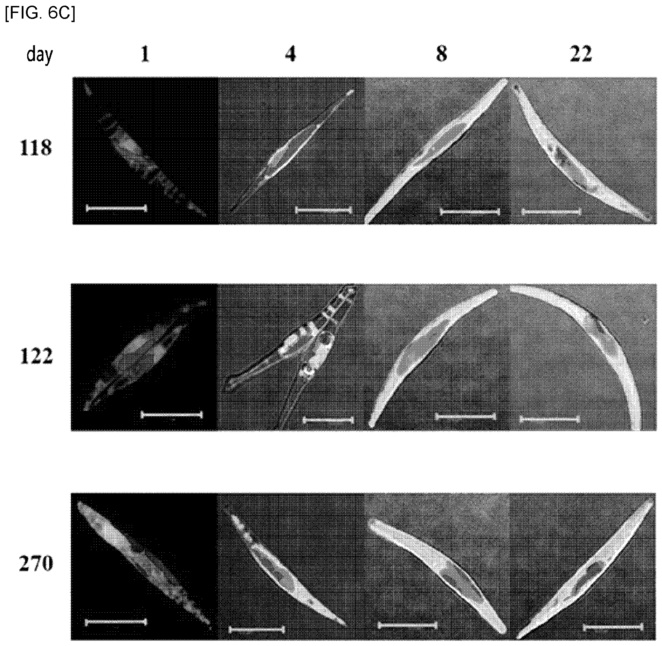

On the contrary, GFP fluorescence in the *HASP1pro:GFP* transgenic line strongly increased throughout the entire cytoplasm from the exponential phase to the early stationary phase and was maintained at high levels throughout the stationary phase (FIG. 6C).

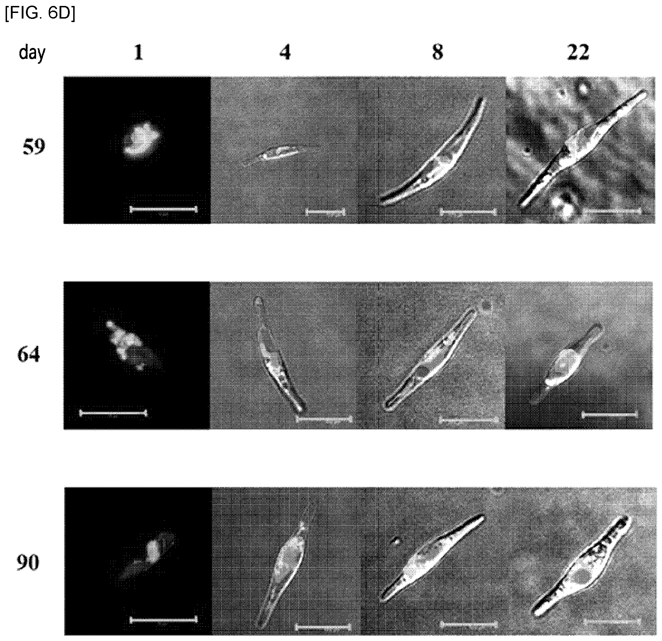

Meanwhile, the signal peptide of the HASP1 protein caused significant changes in subcellular localization of GFP during all growth phases. GFP fluorescence in the *fcpApro:SP-GFP* lines was weakly localized near chloroplast compartment in the exponential phase. However, GFP fluorescence in the *HASP1pro:SP-GFP* lines was rapidly increased from the exponential phase to the early stationary phase (day 8) and maintained at a high level throughout the late stationary phase (FIG. 6D).

These findings suggest that the HASP1 signal peptide efficiently leads the entry of GFP into the protein secretion pathway.

By combining the above-described results, it was confirmed that the *HASP1* promoter and the signal peptide may be used as strong tools for efficiently expressing and secreting recombinant proteins throughout all growth phases of *Phaeodactylum tricomutum.*

The above description of the present invention is provided for the purpose of illustration, and it would be understood by those skilled in the art that various changes and modifications may be made without changing technical conception and essential features of the present invention. Thus, it is clear that the above-described embodiments are illustrative in all aspects and do not limit the present invention. Therefore, the scope of the disclosure is defined not by the detailed description, but by the claims and their equivalents, and all variations within the scope of the claims and their equivalents are to be construed as being included in the disclosure.

## Claims

1. An *HASP1* promoter comprising a polynucleotide of SEQ ID NO: 1.

2. An HASP1 signal peptide expressed by a polynucleotide of SEQ ID NO: 2 or comprising an amino acid sequence of SEQ ID NO: 3.

3. An expression vector comprising a promoter of SEQ ID NO: 1 and/or a signal peptide encoding sequence of SEQ ID NO: 2.

4. A transformant in which the expression vector of claim 3 has been introduced into a host cell.

5. The transformant according to claim 4, wherein the host cell is a microalga.

6. The transformant according to claim 5, wherein the microalga is *Phaeodactylum tricornutum.*

7. A method for producing a protein of interest, the method comprising:
(a) obtaining a culture by culturing the transformant according to any one of claims 4 to 6; and
(b) recovering the protein of interest from the culture.

8. A method for expressing a protein of interest, the method comprising transforming a host cell with the expression vector of claim 3.

9. The method according to claim 8, wherein the host cell is a microalga.

10. The method according to claim 9, wherein the microalga is *Phaeodactylum tricornutum.*

11. A method for increasing an expression level of a protein of interest in a host cell, the method comprising transforming the host cell with the expression vector of claim 3.

12. The method according to claim 11, wherein the host cell is a microalga.

13. The method according to claim 12, wherein the microalga is *Phaeodactylum tricornutum.*

14. A use of the promoter of claim 1 or the signal peptide of claim 2 for increasing an expression level of a protein of interest in a host cell.


<110>     KOREA INSTITUTE OF SCIENCE AND TECHNOLOGY
          ALGAEPRONA INC.

<120>     Novel promoter HASP1 of Phaeodactylum tricornutum and signal
          peptide thereof and uses thereof

<130>     OPA20106-PCT

<150>     KR 10-2019-0079014
<151>     2019-07-01

<160>     12

<170>     KopatentIn 2.0

<210>     1
<211>     499
<212>     DNA
<213>     Phaeodactylum tricornutum

<400>     1
catacagtga atgtaacttt cgaattgaca gtattagtag tcgtattgac agtgaggcac        60

gcccctcaat gtgcgaggtg gaaaatatac cagcatgaca atgaatcttg gagattcttt       120

tgctgtcatc aagattcacc gccaaatctt caggaaccta tcacgtccac aggcgatgtt       180

aattcttgag tcgtcaaaac aaagtcctgt cctacctgta gaagttgaca gcgagcaatt       240

gtatgcaaac ttctgacttt gttataataa cattaaaggt aattaagtat cttcaattag       300

gcattttgtc actgtcagtc cgttccgaca atataggtag atttggaatg aatcttttct       360

atgctgctgc gaatcttgta cacctttgag gccgtagatt ctgtccgacg aagcgataat       420

tattgcaaaa tacatggact cattattttg attcgatttc tttttggtat ccgactcgaa       480

aagatccatc acggcgagc                                                    499


<210>     2
<211>     54
<212>     DNA
<213>     Phaeodactylum tricornutum

<400>     2
accatgaatc ttcgttgtat ccttccgttt ctcctcgcaa gcttctcggc tggg              54


<210>     3
<211>     18
<212>     PRT
<213>     Phaeodactylum tricornutum

<400>     3
Met Asn Leu Arg Cys Ile Leu Pro Phe Leu Leu Ala Ser Phe Ser Ala
  1               5                  10                  15

Gly Ala



<210>     4

<211>    793
<212>    PRT
<213>    Phaeodactylum tricornutum

<400>    4
Met Asn Leu Arg Cys Ile Leu Pro Phe Leu Leu Ala Ser Phe Ser Ala
1               5                   10                  15

Gly Ala Phe Val Phe Asn Arg Val Ala Thr Phe Asn Ile Cys Thr Gln
            20                  25                  30

Ile Asp Pro Thr Cys Asn Thr Asp Thr Glu Ala Val Ala Glu Ile Val
        35                  40                  45

Asp Ala Ser Pro Asp Gly Asn Thr Leu Leu Tyr Thr Asp Gly Val Ala
    50                  55                  60

Gly Asn Ile Gly Leu Val Asp Ile Ser Asp Pro Ser Asn Pro Val Gly
65                  70                  75                  80

Leu Gly Thr Ile Asp Val Gly Gly Glu Pro Thr Ser Val Ala Val Thr
                85                  90                  95

Gly Asp Gly Leu Tyr Ala Val Ala Ala Val Asn Thr Ser Pro Asp Phe
            100                 105                 110

Val Asn Val Thr Gly Asn Leu Ile Val Ile Asp Ile Glu Ser Gln Leu
        115                 120                 125

Ile Val Ala Thr Leu Pro Leu Pro Gly Gln Pro Asp Ser Leu Lys Val
    130                 135                 140

Ser Pro Asp Asn Gln Phe Ile Ala Ile Ala Ile Glu Asn Glu Arg Asp
145                 150                 155                 160

Glu Asp Leu Gly Asp Gly Ala Pro Pro Gln Met Pro Pro Gly Ser Leu
                165                 170                 175

Val Val Val Asp Lys Ser Asp Thr Asp Pro Thr Met Trp Ser Thr Thr
            180                 185                 190

Leu Val Asn Leu Thr Gly Leu Asp Gly Leu Arg Phe Pro Glu Asp Pro
        195                 200                 205

Glu Pro Glu Phe Val Asp Ile Ser Thr Asp Asn Val Cys Val Ile Thr
    210                 215                 220

Leu Gln Glu Asn Asn Gly Ile Val Ile Leu Asp Leu Ala Ser Leu Ser
225                 230                 235                 240

Val Leu Thr Ser Phe Ser Ala Gly Ala Val Ala Glu Ile Thr Gln Ile
                245                 250                 255

Asp Ala Thr Glu Asp Asp Met Ile Ser Gln Thr Glu Ser Leu Asn Asp
            260                 265                 270

Ile Leu Arg Glu Pro Asp Ser Ala Val Phe Ile Gly Thr Asp Tyr Phe
        275                 280                 285

Ala Thr Ala Asp Glu Gly Asp Leu Asp Gly Gly Ser Arg Gly Phe Thr
    290                 295                 300

Ile Phe Gly Leu Asp Gly Thr Val Val Tyr Glu Ser Gly Asn Phe Leu

305                 310                 315                 320

Glu His Ala Thr Ala Arg Tyr Gly His Tyr Pro Glu Gly Arg Ser Glu
                325                 330                 335

Asn Lys Gly Asn Glu Pro Glu Gly Met Thr Tyr Ser Thr Phe Gly Asp
            340                 345                 350

Glu Asn Leu Leu Phe Val Leu Ser Glu Arg Ala Asn Thr Val Tyr Val
        355                 360                 365

Phe Asn Val Asp Pro Thr Asp Pro Ser Asn Val Ser Phe Lys Gln Val
    370                 375                 380

Leu Pro Ser Gly Val Gly Pro Glu Gly Val Thr Ala Ile Pro Ser Arg
385                 390                 395                 400

Asn Leu Leu Ala Val Ala Ser Glu Val Asp Asp Arg Gly Ala Ala Ile
                405                 410                 415

Arg Ser Ser Ile Thr Ile Tyr Glu Tyr Ala Glu Gly Thr Ala Ala Tyr
            420                 425                 430

Pro Thr Leu Val Ser Asn Asn Arg Ala Asp Gly Thr Pro Ile Pro Phe
        435                 440                 445

Ser Ala Leu Ser Gly Leu Ala Ala Ala Asp Ala Pro Gly Ile Gly Gly
    450                 455                 460

Gln Ala Gly Ile Leu Tyr Ser Val Asp Asp Ser Phe Tyr Ser Lys Ser
465                 470                 475                 480

Arg Ile Phe Thr Ile Asp Val Thr Ser Phe Pro Tyr Ala Ile Thr Asn
                485                 490                 495

Glu Met Arg Val Met Asp Ser Asp Gly Val Leu Ala Ala Ala Phe Pro
            500                 505                 510

Asn Ser Thr Leu Val Asn Asp Asp Met Thr Val Asn Leu Asp Gln Glu
        515                 520                 525

Gly Ile Ala Val Ser Arg Glu Gly Gly Tyr Trp Ile Ala Asn Glu Gly
    530                 535                 540

Ala Gly Thr Val Gly Asp Ile Glu Asn Pro Val Thr Thr Ala Asn Phe
545                 550                 555                 560

Val Val Lys Val Ser Thr Glu Gly Val Ile Glu Thr Val Val Thr Leu
                565                 570                 575

Pro Glu Glu Ile Asp Ala Ile Gln Val Arg Phe Gly Phe Glu Gly Ile
            580                 585                 590

Ala Glu Tyr Gly Asp Phe Ile Val Val Ala Phe Gln Arg Ala Trp Gly
        595                 600                 605

Glu Glu Ala Asn Pro Arg Leu Gly Ile Tyr Asp Thr Thr Ser Ser Thr
    610                 615                 620

Trp Lys Phe Val Phe Tyr Pro Leu Asp Thr Pro Ser Ser Gln Asn Gly
625                 630                 635                 640

Gly Trp Val Gly Ile Gly Asp Leu Ser Pro Val Gly Asn Gly Asp Phe

645                 650                 655

Val Val Leu Glu Arg Asp Asn Gln Gly Gly Pro Asp Ala Ala Ile Lys
            660                 665                 670

Lys Ile Tyr Lys Ile Ser Leu Gly Ser Met Leu Glu Val Glu Glu Ser
        675                 680                 685

Thr Thr Val Glu Lys Thr Leu Val Arg Asp Val Leu Pro Asp Leu Ala
    690                 695                 700

Ala Ser Asn Gly Asn Ile Tyr Glu Lys Leu Glu Gly Leu Ala Val Thr
705                 710                 715                 720

Ser Glu Gly Glu Val Trp Ile Asn Asn Asp Asn Asp Gly Val Asp Asp
                725                 730                 735

Asn Ser Gly Glu Gln Gln Leu Ile Ser Leu Gly Met Ile Val Thr Ile
            740                 745                 750

Pro Glu Gly Thr Ala Thr Pro Ile Glu Asp Ser Pro Thr Val Ser Pro
        755                 760                 765

Val Gln Gly Thr Ser Met Ala Asn Arg Val Gly His Gly Gly Ala Leu
    770                 775                 780

Leu Val Phe Gly Phe Phe Ala Leu Leu
785                 790

<210>    5
<211>    28
<212>    DNA
<213>    Artificial Sequence

<220>
<223>    primer

<400>    5
catatgcata cagtgaatgt aactttcg                                          28

<210>    6
<211>    28
<212>    DNA
<213>    Artificial Sequence

<220>
<223>    primer

<400>    6
gaattcgctc gccgtgatgg atcttttc                                          28

<210>    7
<211>    27
<212>    DNA
<213>    Artificial Sequence

<220>
<223>    primer

<400>    7
gctagcatgg tgagcaaggg cgaggag                                          27


<210>    8
<211>    27
<212>    DNA
<213>    Artificial Sequence

<220>
<223>    primer


<400>    8
gaattcatgg tgagcaaggg cgaggag                                          27


<210>    9
<211>    29
<212>    DNA
<213>    Artificial Sequence

<220>
<223>    primer


<400>    9
ggatccttac ttgtacagct cgtccatgc                                        29


<210>    10
<211>    87
<212>    DNA
<213>    Artificial Sequence

<220>
<223>    primer


<400>    10
gaattcacca tgaatcttcg ttgtatcctt ccgtttctcc tcgcaagctt ctcggctggg      60

gctagcatgg tgagcaaggg cgaggag                                          87


<210>    11
<211>    19
<212>    DNA
<213>    Artificial Sequence

<220>
<223>    primer


<400>    11
gtgtcggggc tggcttaac                                                   19


<210>    12
<211>    18
<212>    DNA

<213>    Artificial Sequence

<220>
<223>    primer

<400>    12
gccgcacgtc cctggttg                                                    18